Europäisches Patentamt

European Patent Office

Office européen des brevets

(11) Publication number: 0 354 504
A2

# EUROPEAN PATENT APPLICATION

(21) Application number: 89114505.4

(22) Date of filing: 05.08.89

(51) Int. Cl.⁴ C12N 15/57 , A61K 37/54 , C12P 21/02

Claims for the following Contracting States: ES + GR

The microorganism(s) has (have) been deposited with The Fermentation Research Institute under number(s) FERM BP 1472,1473,1858

(30) Priority: 09.08.88 JP 197144/88

(43) Date of publication of application: 14.02.90 Bulletin 90/07

(84) Designated Contracting States: AT BE CH DE ES FR GB GR IT LI LU NL SE

(71) Applicant: HOECHST JAPAN LIMITED 10-16, 8-chome, Akasaka, Minato-ku Tokyo(JP)

(72) Inventor: Hashimoto, Tamotsu 1-5-10, Sakae-Cho Asaka-Shi Saitama-ken(JP) Inventor: Takahashi, Mikiko 1-11, Ayase Hasuda-shi Saitama-ken(JP)

(74) Representative: Klein, Otto, Dr. et al Hoechst AG Zentrale Patentabteilung Postfach 80 03 20 D-6230 Frankfurt am Main 80(DE)

(54) Hybrid protein C constructs and methods for their preparation.

(57) Hybrid human protein C constructs whose Gla domain are replaced by the Gla domain of bovine protein C are disclosed. The number of γ-carboxylglutamic acid residues is increased from 9 to 11.

The hybrid protein C constructs are produced by culturing host cells transfected with a plasmid vector containing DNA coding for the protein.

EP 0 354 504 A2

# Hybrid Protein C Constructs and Methods for Their Preparation

Detailed Explanation of the Invention

(1) Applied fields in industry:

This invention relates to novel hybrid protein C constructs having anti-blood coagulation activity.

(2) Technological background:

Human protein C is a precursor of a serine protease detected in human plasma.

It is well known that human protein C is activated by a thrombin-thrombomodulin complex. Activated protein C inhibits blood coagulation through inactivation of Factor VIIIa and Factor Va in the presence of calcium ions, or accelerates blood fibrinolysis through inactivation of tissue plasminogen activator inhibitor (hereinafter referred to as "PAI").

The genes coding human and bovine protein C were cloned and sequenced by Foster et al., Proc. Natl. Acad. Sci. USA, 82, 4673-4677, (1985) and by Long et al., Proc. Natl. Acad. Sci. USA, 81, 5653-5656, (1984), respectively.

Human protein C has 9 characteristic glutamic acid residues at the 6th, 7th, 14th, 16th, 19th, 20th, 25th, 26th, and 29th positions in the amino terminal region and bovine protein C has 2 additional characteristic glutamic acid residues at the 23rd and 35th positions. These glutamic acid residues are carboxylated at their $\gamma$-carbon through vitamin K dependent post-translational modification. The domain including these $\gamma$-carboxyl glutamic acid residues (hereinafter referred to as "Gla domain") is required for complex formation in the presence of calcium with negatively charged phospholipids on the cell membrane. The function of the Gla domain was reviewed in Taisha (Metabolism), 19, No. 9 (1982) (in Japanese).

Bovine protein C has similar activities to those of human protein C and the homology of the primary structures between these proteins is known to be very high.

(3) Purpose of the invention:

The purpose of this invention is to supply a novel hybrid protein C constructs made by replacing the Gla domain of human protein C with a corresponding segment of bovine protein C to enhance the calcium binding activity of human protein C by increasing the number of $\gamma$-carboxylglutamic acid residue from 9 to 11, then to improve its activation or protein C activity itself.

(4) Construction of the invention:

The present invention is directed to hybrid proteins constructed by replacing the Gla domain of human protein C with the Gla domain of bovine protein C or with its equivalent.

The amino acid sequences near the amino terminal of human protein C and bovine protein C are listed in Table 1.

The amino acid residues in Table 1 are abbreviated to the following:
A, Ala; N, Asn; S, Ser; F, Phe; L, Leu; E, Glu; R, Arg; H, His; C, Cys; I, Ile; D, Asp; K, Lys; Q, Gln; V, Val; P, Pro; T, Thr; W, Trp; M, Met; G, Gly; Y, Tyr The glutamic acid residues, designated as E in this table are $\gamma$-carboxylated.

In this invention, the amino acid sequence including the Gla domain indicates the amino acid sequence from the 1st to 43rd residues. To replace the Gla domain of human protein C with the Gla domain of bovine protein C, the corresponding segments in the alignment shown in Table 1 are substituted.

The hybrid proteins in this invention can be produced by genetic engineering techniques. Therefore, this invention is also concerned with the DNA coding for these hybrid proteins and the method of producing these hybrid proteins through genetic engineering techniques.

The gene coding for hybrid proteins in this invention can be constructed by replacing the nucleotide sequence coding the Gla domain of human protein C with the nucleotide sequence coding the Gla domain of bovine protein C. The prepro sequence of human protein C can be additionally replaced with the prepro sequence of bovine protein C. The prepro sequence can be also replaced with the prepro sequence of vitamin K dependent blood coagulation proteins, such as Factor X.

The genes constructed by the above methods can be expressed in the proper host cells transformed by the proper expression vectors constructed by ligating the protein coding sequence with a promoter, a terminator. etc. by the commonly used methods in this field. Preferable hosts are eukaryotic cells, which can modify the recombinant proteins in post-translational manners, e.g., glycosylation, $\gamma$-carboxylation or $\beta$-hydroxylation.

Examples for host cells favorable for producing are animal cells such as CHO or BHK cells. The construction of recombinant genes, the additional ligation of functional regions necessary for efficient expression, transfection to host cells, expression of recombinant products in host cells, and isolation of recombinant products, etc. can be carried out by any methods generally available in this field.

(5) Effect of the invention:

Hybrid proteins of this invention have an increased number of glutamic residues (from 9 to 11) by changing the Gla domain from the human type to the bovine type. Through the increase of the number of $\gamma$-carboxylation sites, the improved effects are obtained.

(6) Explanation by examples:

The invention is explained by examples in the following. In the examples, the gene constructed by replacing the Gla domain and the prepro sequence of human protein C with the Gla domain of bovine protein C and the prepro sequence of human Factor X was used. The DNA sequence coding Factor X was described in Biochemistry, 25, 5098 (1986).

In the following examples, the gene encoding human protein C described in Japanese Patent Application No. 96341 87 (Laid-Open Unexamined Publication No. 263083/88) was used, as well as the expression vector, including the above mentioned gene, and the new manufacturing process through gene engineering techniques described in Japanese Patent Application No. 96340/87 (Laid-Open Unexamined Publication No. 267288/88). These methods are explained as reference examples. However the examples and reference examples do not limit the invention.

Reference example 1 (construction of pCs4)

Plasmid pPCl including the gene coding protein C was constructed by the inventors et al. described in Japanese Laid-Open Unexamined Patent Publication No. 263083/88. The plasmid was transformed into E. coli K-12/Om225 which was deposited with the Fermentation Research Institute, Agency of Industrial Science and Technology as FERM BP-1858.

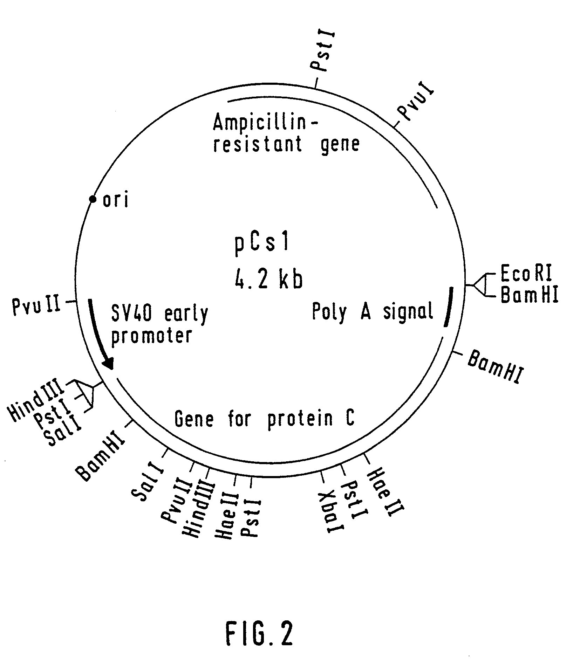

Furthermore, an expression vector pCs1 was constructed from the gene encoding human protein C and the SV40 early promoter. The plasmid was transformed into E. coli K-12/Om225 which was deposited with the Fermentation Research Institute as FERM BP-1473. The restriction map is shown in Fig. 2.

pCs4 was constructed from pCs1 as described in Japanese Laid-Open Unexamined Patent Publication No. 267288/88. pCs4 has 2 BstXI sites upstream from the SV40 early promoter and downstream from the poly A signal. The BstXI site was devised to produce the following asymmetric cohesive ends by digestion with BstXI.

$$5' \cdots \cdots CTGG \boxed{\text{Gene}} CCACGGGG \cdot 3'$$
$$3' \cdot CCCCGACC \boxed{\phantom{Gene}} GGTG \cdots \cdots 5'$$

The fragments link with each other necessarily in tandem. This makes it possible to prepare DNA comprising many tandem repeats of expression unit of protein C (a promoter plus a protein C gene plus a poly A signal). Protein C is produced with high efficiency by cultivating host animal cells transfected with the DNA comprising many tandem repeats.

Production process of pCs4 is as follows: the fragment obtained by digesting pCs1 with EcoRI was ligated with the double-stranded oligonucleotide synthesized chemically as mentioned below.

$$\begin{array}{c} \downarrow \\ 5'\ pAATTG\ \boxed{CCA}\ CGGGGC\ \boxed{TGG}\ C\cdots\cdots 3' \\ 3'\ \cdots\cdots C\ \boxed{GGT}\ GCCCG\ \boxed{ACC}\ GAGCTp\ 5' \\ \uparrow \end{array}$$

wherein P represents the phosphate group coupled at the 5' ends for the ligation, □ indicates the BstXI recognition site, and ↑ indicates the cleavage site by the said enzyme.

The left side of the oligonucleotide is arranged so as to be a 5' protruding sequence which can be ligated to the cohesive end of EcoRI-cleaved pCs1, which does not regenerate the EcoRI site. This arrangement is made so that the EcoRI site produced in the subsequent step will be a unique site. The right side is the portion to be linked with the XhoI cohesive end.

The ligation product was digested with XhoI followed again by ligation. Both ends of pCs1 cleaved with EcoRI were linked respectively with one molecule of the synthetic oligonucleotide. Both ends of the product were subjected to ligation (wherein the XhoI site is formed) to form a circular plasmid.

Then, the resulting plasmid was partially digested with PvuII. As there were two PvuII sites in pCs1, the cleavage took place at both, either one or none of the sites to give a mixture. Accordingly, the mixture was subjected to size fractionation by means of agarose gel electrophoresis to isolate the product in which the PvuII site located upstream of the SV40 early promoter only had been cleaved. The isolated DNA chain was ligated with a chemically synthesized double-stranded oligonucleotide with the chemical structure shown below.

$$\begin{array}{c} \downarrow \\ 5'\ pTT\ \boxed{CCA}\ GCCCCG\ \boxed{TGG}\ \cdots\cdots 3' \\ 3'\ \cdot AA\ \boxed{GGT}\ CGGGGC\ \boxed{ACC}\ TTAAp\ 5' \\ \uparrow \end{array}$$

wherein symbols are as defined above, and the right side is the portion to be linked with the section cleaved by EcoRI.

The left side of the oligonucleotide was linked with the PvuII site of the cleaved plasmid DNA, which did not regenerate the PvuII site. The ligation product was digested with EcoRI followed again by ligation. Both ends of the PvuII-cleaved plasmid DNA were linked with one molecule of the synthetic oligonucleotide respectively. Both ends of the product were subjected to ligation (wherein an EcoRI site is located) to form a circular plasmid. The resulting plasmid was cleaved with XhoI and EcoRI, and the fragment was cloned into pHSG396 having a chloramphenicol-resistant marker (available from Takara Shuzo Co., Ltd.) cleaved with XhoI and EcoRI. The chloramphenicol-resistant protein C expression vector plasmid thus obtained was named pCs4.

Example 1

Synthesis of a DNA fragment coding for an amino acid sequence including the Gla domain of bovine protein C and the prepro sequence of human Factor X

A DNA fragment, the sequence of which is shown in Table 2, was synthesized so as to have a SalI site for convenience of ligation and an EcoRI site and to cover (a) the 40 amino acid residues of the prepro sequence of Factor X, (b) the 1st to 43rd amino acid residues from the N-terminus of bovine protein C following the prepro sequence, and (c) the nucleotide sequence coding for the 44th to 46th amino acid residues of human protein C. The adenine just before the start ATG was selected to expect a preferable effect on expression of hybrid proteins. And the nucleotide sequence coding for the 44th to 46th amino acid residues of protein C was introduced to create a new SalI site to ligate the DNA fragment with the human protein C gene.

Eight oligonucleotides to yield four DNA fragments separated with HindIII, XbaI, and BglII, shown in Table 2, were synthesized by a DNA synthesizer, model 380A (Applied Biosystem Co., U.S.A.). After anealing, 4 double-strand fragments were ligated by T₄ DNA ligase at the HindIII, XbaI and BglII sites, respectively. The ligated DNA fragment flanked by SalI sites was inserted into pUC18, commercially available from Takara Shuzo Co., Ltd., and amplified in E. coli K-12/HB101. The amplified vector was digested by SalI, and the synthetic DNA fragment was obtained by ordinary purification methods.

Example 2

Construction of expression vectors for hybrid proteins

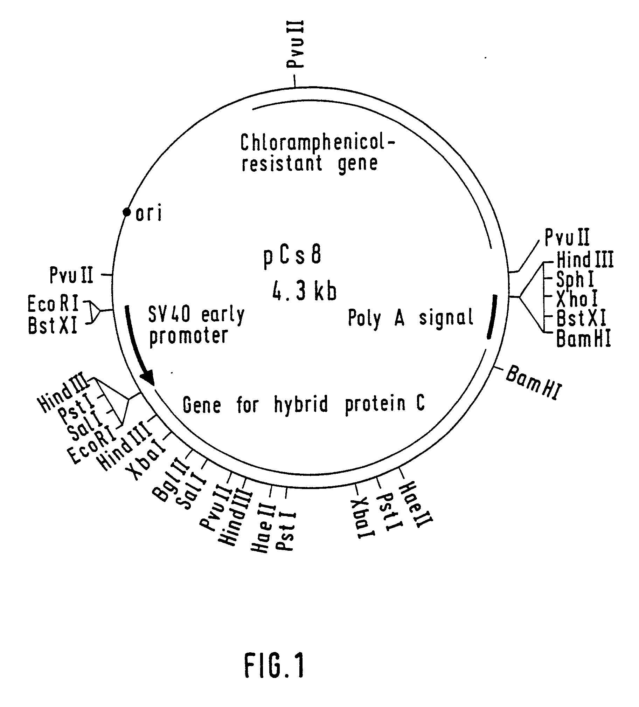

The gene coding for part of protein C could be removed from pCs4 by SalI digestion at the SalI site just upstream of leader sequence-coding sequence and at the SalI site corresponding to the 45th valine residue and 46th aspartic acid residue of protein C. The synthetic DNA fragment synthesized in Example 1 was inserted into pCs4 between these SalI sites, and introduced into E. coli K-12/HB101. The transformants harbouring a plasmid in an appropriate orientation, named pCs8, were screened and cultivated. pCs8 had the nucleotide sequence, shown in Table 3, of the hybrid protein. Its restriction map is shown in Fig. 1.

Reference example 2 (construction of pHSG293)

pHSG293 was also constructed which had a neo gene as a selection marker and BstXI site providing the same asymmetric cohesive ends as pCs8 by digestion as described in Japanese Laid-Open Unexamined Patent Publication No. 267288/88. The fragment obtained by digesting pHSG293 with BstXI was ligated with the fragment obtained by digesting pCs8 so that the selection of transfectants was possible. A production process is as follows: when pHSG274 deposited as ATCC 37301, a cosmid vector with the neo gene which confers kanamycin resistance to E. coli host and G418 (geneticin) resistance in eucaryotic cells was digested with BstXI, cleavage took place as shown in the following flow sheet.

pHSG274
↓ BstXI

```
 CCA  TCATG          A  TGG
 GGT  A             GTACT  ACC  ···· HindIII
cos
```

Synthetic
Oligonucleotide GTAC | CCA GCCCCG TGG | −XbaI−lacOP− A
                       GGT CGGGGC ACC                  TTCGA
   (BstXI⁻)              BstXI              (HindIII)

↓ Ligation

```
  BstXI⁻ − CCA GCCCCG TGG −XbaI−lacOP− A
            GGT CGGGGC ACC              TTCGA
             BstXI                    (HindIII)
cos
                          A  TGG
                         GTACT ACC ····· HindIII
```

↓ HindIII

```
  BstXI⁻ − CCA GCCCCG TGG −XbaI−lacOP− A
            GGT CGGGGC ACC              TTCGA
             BstXI                    (HindIII)
cos
                                      (HindIII)
```

↓ Ligation

pHSG 293

↓ BstXI

3.6kb fragment

Then, the digested fragment was ligated with the synthesized oligonucleotide shown in the flow sheet and the BstXI site was destroyed. Subsequent digestion of the cleavage product with HindIII results in removal of the HindIII-BstXI portion contained in the cosmid vector and the excessive synthetic fragments repeatedly ligated. The resulting product was subjected to ligation to provide a circular plasmid in which the left side of the synthetic oligonucleotide was linked with the HindIII site of the cosmid vector.

The XbaI site in the synthetic oligonucleotide sequence was introduced to distinguish pHSG274 and pHSG293 from each other. The lac operator in the synthetic oligonucleotide was introduced to select transformants by kanamycin resistance after cosmid-packaging and to distinguish transformants that form

EP 0 354 504 A2

blue colonies in the presence of X-gal.

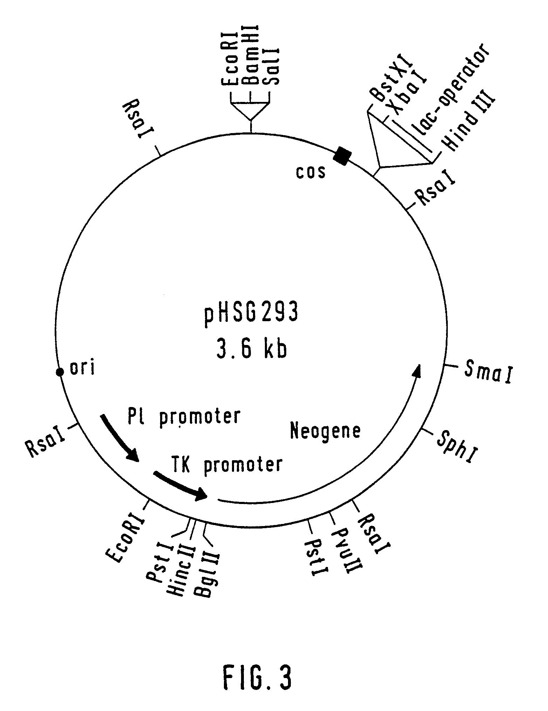

The plasmid was named pHSG293. Its restriction map is shown in Fig. 3.

In the following example, the constructed gene units derived from pCs8 for expression of a hybrid protein and the neo gene removed from pHSG293 were multiply-linked to the same orientation by using uni-directed cohesive ends of BstXI.

Then, these multiple linked genes were in vitro packaged in phage particles and transfected into E. coli cells. The transfectants were screened to obtain clones harbouring desired recombinant cosmid DNAs through kanamycin resistance derived from the neo gene. Then, the recombinant cosmid DNAs obtained by the above method were introduced into CHO cells by a standard method available in this field. Again, G418-resistant CHO cells derived from the neo gene were screened, which efficiently produced desired hybrid proteins.

Example 3

Production of hybrid proteins in animal cells and confirmation of its protein C activities

After digesting pCs8, prepared in Example 2, by BstXI, a 2.0 Kbp DNA fragment was isolated by agarose gel electrophoresis. This 2.0 Kbp DNA and BstXI-digested pHSG293 were ligated at a molar ratio of 20 and 1, respectively. This ligated DNA was packaged in vitro by lamda phage packaging mixture commercially available from Takara Shuzo co.. Ltd. Then packaged recombinant lamda genomes were transfected into E. coli K-12/Om206, deposited as FERM BP-1472 in the Fermentation Research Institute. The transfectants were cultivated under the condition to select kanamycin resistants. The colonies harbouring high copy numbers of genes coding for a hybrid protein were chosen and cultivated to isolate circular cosmid DNAs.

These circular cosmid DNAs were introduced into CHO cells by the calcium phosphate method. Then G418-resistant transfectants derived from pHSG293 were screened and cultured in MEMα medium, commecially available from Gibco Laboratories Inc., including 10% FCS, purchased from Gibco Laboratories Inc., and 0.1 μg/ml vitamin $K_3$. About 1 x $10^6$ transfectants were seeded into a 6 cm-∅ petri dish and cultivated at 37°C overnight. After exchanging 25 the overnight medium for fresh medium, the cells were additionally cultivated for at least 24 hours. After collecting the culture supernatant, the amount of hybrid protein C in the medium was assayed by ELISA using ananti-human protein C antibody preparation.

Consequently, 2 out of 24 clones were confirmed to produce 432 ng/ml or 274 ng/ml of immunoreactive hybrid protein C in the medium. The best colony was further cultivated on a larger scale.

Example 4

Purification of a hybrid protein C

The hybrid protein C was purified from 236 ml of the culture supernatant of highly producing cells obtained in Example 3.

To the supernatant, one-third volume of cold secondary distilled water and one-twentieth volume of 1M imidazole-HCl buffer solution (pH 6.5) was added. The mixture was applied to QAE-Sepharose Fast Flow column (about 10-ml volume), purchased from Pharmacia Co. The column was washed with 0.11M NaCl, 50 mM imidazole-HCl buffer solution (pH 6.5) until no proteins were detected in the eluate. Bound hybrid protein C was, then, eluted with 0.5 M NaCl, 50 mM imidazole-HCl buffer solution (pH 6.5). $CaCl_2$ was added to the eluate to a final 5 mM concentration. Again, the eluate was applied to a calcium-dependent anti-human protein C antibody column, prepared from an activated CH-Sepharose CL-4B purchased from Pharmacia Co. The bound hybrid protein C was eluted with TBS buffer solution (0.15 M NaCl, 20 mM Tris-HCl, pH 7.2) including 5 mM EDTA. Finally, 134 μg of purified hybrid protein C was obtained.

Example 5

7

### Activity of prified hybrid protein C

The chromogenic activity of the purified hybrid protein C obtained in Examle 4 was determined. Fourty $\mu l$ of the eluate (protein concentration: 23 $\mu g/ml$) and 40 $\mu l$ of the reaction buffer solution (2.5 $\mu g/ml$ BSA, 5 mM EDTA, 50 mM Tris-HCl, pH. 8.0) were mixed with 20 $\mu l$ of Protac® (1U/ml, purchased from Pentafarm Co., switzerland) and incubated for 10 min at 37°C.

Chromozyme PCa, a synthetic substrate purchased from Boehringer-Mannheim-Yamanouchi Co., was added to the reaction mixture and the volume of the solution was adjusted to 500 $\mu l$. The mixture was further incubated for 5 min at 37°C. The reaction was terminated by adding 400 $\mu l$ of 50% acetate and the amount of the reaction product was determined by the absorbance of the solution at 405 nm. When compared with purified protein C derived from human blood, the chromogenic activity of the hybrid protein C was 172%.

### Explanation of Tables

Table 1 shows alignments of the amino acid sequence near the amino terminus of bovine protein C and human protein C.

Table 2 shows the nucleotide sequence of synthetic DNA coding for the amino acid sequecne including the prepro sequence of human Factor X and the Gla domain of bovine protein C.

Table 3 shows the nucleotide sequence coding for hybrid protein C constructed by replacing the Gla domain and the prepro sequence of human protein C by the Gla domain of bovine protein C and the prepro sequence of human Factor X, respectively, and the amino acid sequence of the hybrid protein C.

Table 1

| | 10 | 20 | 30 | 40 |
|---|---|---|---|---|
| Bobine protein c | ANSFLEELRPGNVERECSEEVCEFEEAREIFQNTEDTMAFWSKH |
| Human protein C | ANSFLEELRHSSLERECIEEICDFEEAKEIFQNVDDTLAFWSKH |

## Table 2

→ **Prepro sequence of Factor X**

<u>GTCGACGAAT TC</u>AATGGGGC GCCCACTGCA CCTCGTCCTG CTCAGTGCCT CCCTGGCTGG

**Sa l l    EcoR l**


CCTCCTGCTG CTCGGGGA<u>AA GCTT</u>GTTCAT CCGCAGGGAG CAGGCCAACA ACATCCTGGC

**H l n d Ⅲ**


→ **1st to 43 rd amino acid residues of bovine protein C**

GAGGGTCACG AGGGCCAATT CCTT<u>TCTAGA</u> GGAGCTGCGG CCCGGCAACG TGGAGCGTGA

**Xba l**


GTGCTCAGAG GAGGTCTGTG AGTTCGAGGA AGCTCGGG<u>AG ATCT</u>TCCAAA ACACGGAAGA

**B g l Ⅱ**


→ **44th to 46th amino acid residues of human protein C**

CACAATGGCC TTCTGGTCCA AGCAC<u>GTCGA C</u>

**Sa l l**

EP 0 354 504 A2

## Table 3-1

```
*                                                                              *
ATG.GGG.CGC.CCA.CTG.CAC.CTC.GTC.CTG.CTC.AGT.GCC.TCC.CTG.GCT.GGC.CTC.CTG.CTG.CTC
Met-Gly-Arg-Pro-Leu-His-Leu-Val-Leu-Leu-Ser-Ala-Ser-Leu-Ala-Gly-Leu-Leu-Leu-Leu

*                                                                              *
GGG.GAA.AGC.TTG.TTC.ATC.CGC.AGG.GAG.CAG.GCC.AAC.AAC.ATC.CTG.GCG.AGG.GTC.ACG.AGG
Gly-Glu-Ser-Leu-Phe-Ile-Arg-Arg-Glu-Gln-Ala-Asn-Asn-Ile-Leu-Ala-Arg-Val-Thr-Arg

*                                                                              *
GCC.AAT.TCC.TTT.CTA.GAG.GAG.CTG.CGG.CCC.GGC.AAC.GTG.GAG.CGT.GAG.TGC.TCA.GAG.GAG
Ala-Asn-Ser-Phe-Leu-Glu-Glu-Leu-Arg-Pro-Gly-Asn-Val-Glu-Arg-Glu-Cys-Ser-Glu-Glu

*                                                                              *
GTC.TGT.GAG.TTC.GAG.GAA.GCT.CGG.GAG.ATC.TTC.CAA.AAC.ACG.GAA.GAC.ACA.ATG.GCC.TTC
Val-Cys-Glu-Phe-Glu-Glu-Ala-Arg-Glu-Ile-Phe-Gln-Asn-Thr-Glu-Asp-Thr-Met-Ala-Phe

*                                                                              *
TGG.TCC.AAG.CAC.GTC.GAC.GGT.GAC.CAG.TGC.TTG.GTC.TTG.CCC.TTG.GAG.CAC.CCG.TGC.GCC
Trp-Ser-Lys-His-Val-Asp-Gly-Asp-Gln-Cys-Leu-Val-Leu-Pro-Leu-Glu-His-Pro-Cys-Ala

*                                                                              *
AGC.CTG.TGC.TGC.GGG.CAC.GGC.ACG.TGC.ATC.GAT.GGC.ATC.GGC.AGC.TTC.AGC.TGC.GAC.TGC
Ser-Leu-Cys-Cys-Gly-His-Gly-Thr-Cys-Ile-Asp-Gly-Ile-Gly-Ser-Phe-Ser-Cys-Asp-Cys

*                                                                              *
CGC.AGC.GGC.TGG.GAG.GGC.CGC.TTC.TGC.CAG.CGC.GAG.GTA.AGC.TTC.CTC.AAT.TGC.TCT.CTG
Arg-Ser-Gly-Trp-Glu-Gly-Arg-Phe-Cys-Gln-Arg-Glu-Val-Ser-Phe-Leu-Asn-Cys-Ser-Leu

*                                                                              *
GAC.AAC.GGC.GGC.TGC.ACG.CAT.TAC.TGC.CTA.GAG.GAG.GTG.GGC.TGG.CGG.CGC.TGT.AGC.TGT
Asp-Asn-Gly-Gly-Cys-Thr-His-Tyr-Cys-Leu-Glu-Glu-Val-Gly-Trp-Arg-Arg-Cys-Ser-Cys
```

EP 0 354 504 A2

Table 3-2

*
GCG.CCT.GGC.TAC.AAG.CTG.GGG.GAC.GAC.CTC.CTG.CAG.TGT.CAC.CCC.GCA.GTG.AAG.TTC.CCT
Ala-Pro-Gly-Tyr-Lys-Leu-Gly-Asp-Asp-Leu-Leu-Gln-Cys-His-Pro-Ala-Val-Lys-Phe-Pro

*
TGT.GGG.AGG.CCC.TGG.AAG.CGG.ATG.GAG.AAG.AAG.AGA.TCT.CAC.CTG.AAA.CGA.GAC.ACA.GAA
Cys-Gly-Arg-Pro-Trp-Lys-Arg-Met-Glu-Lys-Lys-Arg-Ser-His-Leu-Lys-Arg-Asp-Thr-Glu

*
GAC.CAA.GAA.GAC.CAA.GTA.GAT.CCG.CGG.CTC.ATT.GAT.GGG.AAG.ATG.ACC.AGG.CGG.GGA.GAC
Asp-Gln-Glu-Asp-Gln-Val-Asp-Pro-Arg-Leu-Ile-Asp-Gly-Lys-Met-Thr-Arg-Arg-Gly-Asp

*
AGC.CCC.TGG.CAG.GTG.GTC.CTT.CTA.GAC.TCA.AAG.AAG.AAG.CTG.GCC.TGC.GGG.GCA.GTG.CTC
Ser-Pro-Trp-Gln-Val-Val-Leu-Leu-Asp-Ser-Lys-Lys-Lys-Leu-Ala-Cys-Gly-Ala-Val-Leu

*
ATC.CAC.CCC.TCC.TGG.GTG.CTG.ACT.GCA.GCC.CAC.TGC.ATG.GAT.GAG.TCC.AAG.AAG.CTC.CTT
Ile-His-Pro-Ser-Trp-Val-Leu-Thr-Ala-Ala-His-Cys-Met-Asp-Glu-Ser-Lys-Lys-Leu-Leu

*
GTC.AGG.CTT.GGA.GAG.TAT.GAC.CTG.CGG.CGC.TGG.GAG.AAG.TGG.GAG.CTG.GAC.CTG.GAC.ATC
Val-Arg-Leu-Gly-Glu-Tyr-Asp-Leu-Arg-Arg-Trp-Glu-Lys-Trp-Glu-Leu-Asp-Leu-Asp-Ile

*
AAG.GAG.GTC.TTC.GTC.CAC.CCC.AAC.TAC.AGC.AAG.AGC.ACC.ACC.GAC.AAT.GAC.ATC.GCA.CTG
Lys-Glu-Val-Phe-Val-His-Pro-Asn-Tyr-Ser-Lys-Ser-Thr-Thr-Asp-Asn-Asp-Ile-Ala-Leu

*
CTG.CAC.CTG.GCC.CAG.CCC.GCC.ACC.CTC.TCG.CAG.ACC.ATA.GTG.CCC.ATC.TGC.CTC.CCG.GAC
Leu-His-Leu-Ala-Gln-Pro-Ala-Thr-Leu-Ser-Gln-Thr-Ile-Val-Pro-Ile-Cys-Leu-Pro-Asp

## Table 3-3

```
*
AGC.GGC.CTT.GCA.GAG.CGC.GAG.CTC.AAT.CAG.GCC.GGC.CAG.GAG.ACC.CTC.GTG.ACG.GGC.TGG
Ser-Gly-Leu-Ala-Glu-Arg-Glu-Leu-Asn-Gln-Ala-Gly-Gln-Glu-Thr-Leu-Val-Thr-Gly-Trp

*
GGC.TAC.CAC.AGC.AGC.CGA.GAG.AAG.GAG.GCC.AAG.AGA.AAC.CGC.ACC.TTC.GTC.CTC.AAC.TTC
Gly-Tyr-His-Ser-Ser-Arg-Glu-Lys-Glu-Ala-Lys-Arg-Asn-Arg-Thr-Phe-Val-Leu-Asn-Phe

*
ATC.AAG.ATT.CCC.GTG.GTC.CCG.CAC.AAT.GAG.TGC.AGC.GAG.GTC.ATG.AGC.AAC.ATG.GTG.TCT
Ile-Lys-Ile-Pro-Val-Val-Pro-His-Asn-Glu-Cys-Ser-Glu-Val-Met-Ser-Asn-Met-Val-Ser

*
GAG.AAC.ATG.CTG.TGT.GCG.GGC.ATC.CTC.GGG.GAC.CGG.CAG.GAT.GCC.TGC.GAG.GGC.GAC.AGT
Glu-Asn-Met-Leu-Cys-Ala-Gly-Ile-Leu-Gly-Asp-Arg-Gln-Asp-Ala-Cys-Glu-Gly-Asp-Ser

*
GGG.GGG.CCC.ATG.GTC.GCC.TCC.TTC.CAC.GGC.ACC.TGG.TTC.CTG.GTG.GGC.CTG.GTG.AGC.TGG
Gly-Gly-Pro-Met-Val-Ala-Ser-Phe-His-Gly-Thr-Trp-Phe-Leu-Val-Gly-Leu-Val-Ser-Trp

*
GGT.GAG.GGC.TGT.GGG.CTC.CTT.CAC.AAC.TAC.GGC.GTT.TAC.ACC.AAA.GTC.AGC.CGC.TAC.CTC
Gly-Glu-Gly-Cys-Gly-Leu-Leu-His-Asn-Tyr-Gly-Val-Tyr-Thr-Lys-Val-Ser-Arg-Tyr-Leu

*
GAC.TGG.ATC.CAT.GGG.CAC.ATC.AGA.GAC.AAG.GAA.GCC.CCC.CAG.AAG.AGC.TGG.GCA.CCT.TAG
Asp-Trp-Ile-His-Gly-His-Ile-Arg-Asp-Lys-Glu-Ala-Pro-Gln-Lys-Ser-Trp-Ala-Pro-***
                                                                              , '

*
TAA.
***-
```

Fig. 1 is a restriction map of pCs8.
Fig. 2 is a restriction map of pCs1.
Fig. 3 is a restriction map of pHSG293.

Claims

1. A derivative of human protein C, characterized in that its aminoterminal region having γ-carboxylated glutamic acid residues (Gla domain) is replaced by the Gla domain of bovine protein C or by an equivalent of this bovine protein sequence with respect to its calcium binding activity and/or its enhanced protein C activity.

2. A protein according to claim 1, characterized in that its first 43 amino acids are those of bovine protein C.

3. DNA coding for a protein according to claim 1 or 2.

4. A gene structure containing a DNA according to claim 3.

5. A host cell containing a gene structure according to claim 4.

6. A method for producing a protein according to claim 1 or 2, characterized by expressing in a host cell a gene structure according to claim 4.

7. A medicament comprising a protein according to claim 1 or 2.

8. A protein according to claim 1 or 2 or a protein obtainable in a method according to claim 6 for use in therapy.

The use of a protein according to claim 1 or 2 for the manufacture of a medicament for treating blood coagulation disorders.

Claims for the following Contracting State: GR

1. A process for the preparation of a derivative of human protein C, whose aminoterminal region having γ-carboxylated glutamic acid residues (Gla domain) is replaced by the Gla domain of bovine protein C or by an equivalent of this bovine protein sequence with respect to its calcium binding activity and/or its enhanced protein C activity.

2. A process according to claim 1, the DNA comprises codons for the first 43 amino acids of bovine protein C.

3. DNA coding for a protein according to claim 1 or 2.

4. A gene structure containing a DNA according to claim 3.

5. A host all containing a gene structure according to claim 4.

6. The use of a protein obtainable by a process according to claim 1 or 2, for the manufacture of a medicament for treating blood coagulation disorders.

Claims for the following Contracting State: ES

1. A process for the preparation of a derivative of human protein C, whose aminoterminal region having γ-carboxylated glutamic acid residues (Gla domain) is replaced by the Gla domain of bovine protein C or by an equivalent of this bovine protein sequence with respect to its calcium binding activity and/or its enhanced protein C activity.

2. A process according to claim 1, the DNA comprises codons for the first 43 amino acids of bovine protein C.

3. The use of a protein obtainable by a process according to claim 1 or 2, for the manufacture of a medicament for treating blood coagulation disorders.

FIG.1

FIG. 2

FIG. 3